Europäisches Patentamt

European Patent Office

Office européen des brevets

(19)

(11) Publication number: **0 135 279**
**A2**

(12) **EUROPEAN PATENT APPLICATION**

(21) Application number: **84304753.1**

(22) Date of filing: **11.07.84**

(51) Int. Cl.⁴: **G 01 N 33/68**
**//C12N15/00**

(30) Priority: **14.07.83 US 513666**

(43) Date of publication of application:
**27.03.85 Bulletin 85/13**

(84) Designated Contracting States:
**AT BE CH DE FR GB IT LI LU NL SE**

(71) Applicant: **Orgenics Ltd.**
**P.O. Box 360**
**Yavne 70650(IL)**

(72) Inventor: **Herzberg, Max**

**Moshav Sataria 73272(IL)**

(74) Representative: **Clifford, Frederick Alan**
**MARKS & CLERK 57/60 Lincoln's Inn Fields**
**London WC2A 3LS(GB)**

(54) Educational kits and process for illustrating biochemical processes.

(57) Method and educational kit for illustrating the process of protein synthesis which is not radioactive-based and uses, for example, enzymatically-induced color reactions.

Educational kit and method for demonstrating the particular codons used to produce a particular amino acid sequence without the use of radioactive-based indicator materials.

EP 0 135 279 A2

## BACKGROUND OF THE INVENTION

### 1. Field of the Invention

The invention relates to educational kits which are used to illustrate relatively sophisticated biochemical processes to high school and college students without the use of radioactive materials which are normally used as indicators in such processes. The invention further relates to methods for performing certain structured biochemical processes on a laboratory scale in a manner which assists students of biochemistry, on both a high school and university level, in understanding and visually observing certain fundamental biochemical principles.

### 2. Description of Prior Art and Background

The past several years have seen an intense interest on both a high school and university level in the biological sciences. Bio-engineering in particular is becoming one of the fastest growing scientific and technological fields. While molecular biology may be taught from textbooks and with the use of relatively crude laboratory experimentation on a high school and college level, efforts at providing more sophisticated laboratory teaching techniques have suffered by virtue of the common use of radionuclides as the standard labelling or tagging agents which are used to follow the progress of various biochemical reactions. Thus, traditionally, protein synthesis is demonstrated by the use of artificial polynucleotides. In such systems, a cell-free system such as, for example, Micrococcus-treated reticulocyte lysate is incubated together with a source of polynucleotide to synthesize a given polypeptide. Thus, for example, incubation of polyuridilic acid with the lysate results in the synthetic formation of polyphenylalanine. Using presently available techniques, the demonstration of this synthesis to the student is achieved through the use of a radioactive precursor monomeric nucleotide such as $H^3$-phenylalanine. Using further precipitation by means of a trichloracetic treatment, the radioactive product made by the synthesis is identified by its radioactivity in a scintillation counter.

However, by virtue of the fact that radioactive materials are used, such techniques and demonstrations are generally not available for general use by students until they have reached an advanced stage in their studies and are well familiar with laboratory procedures, and particularly procedures for safely handling radioactive materials. Likewise, high schools and universities are understandably reluctant to introduce the use of radioactive tracers in their laboratories, as well as to expend the funds to obtain the large and expensive equipment which is necessary for the analysis of such products.

## SUMMARY OF THE INVENTION

It is, therefore, an object of the invention to provide an experimental kit for use in laboratories as well as experimental techniques which simply and clearly illustrate basic biochemical processes without the use of radioactive materials, as well as the heavy and costly equipment associated with the handling and use of radioactive materials.

The above objects are achieved according to the invention by providing an educational kit for illustrating the process of protein synthesis which is not radioactive-based. The kit includes means for forming a homopolypeptide from a nucleotide source and means for complexing the homopolypeptide with an anti-homopolypeptide antibody provided from an antibody source. The anti-homopolypeptides used in this kit may be polyclonal or monoclonal antibodies. The use of monoclonal antibodies eliminates intermediate washing steps. Means are additionally provided for complexing the homopolypeptide with an enzyme capable of inducing a color reaction. This complexing may be performed by means of an intermediate antibody. The enzyme reaction may be either directly with the antibody, or, by means of a number of known enzyme techniques in which one or a series of intermediate antibodies are used with the enzyme itself automatically ultimately being complexed with one of the antibodies. The enzyme is then used in a conventional manner to produce a color change of a dye. An enzyme such as horseradish peroxidase may be used.

In a like manner, the inventive method of illustrating the process of protein synthesis without using radioactive materials includes the steps of forming a homopolypeptide from a nucleotide source and complexing the homopolypeptide with an anti-homopolypeptide antibody from an antibody source. Subsequently, the homopolypeptide is reacted with an enzyme capable of inducing a color reaction. Once again, the enzyme itself may be coupled directly to the antibody or may be coupled by means of intermediate antibodies to the product. By means of the enzyme, color changes in associated dyes can be observed.

According to another aspect of the invention, an educational kit is also provided which demonstrates the particular codons which are used to produce a product (poly)peptide containing a particular amino acid sequence. The kit includes a source of mRNA-free reticulocyte lysate and a source of codon-containing polynucleotide which comprises at least one, and preferably at least three, codon(s) which code for the particular amino acid sequence. Means are provided for incubating a mixture of the source materials to form a product (poly)peptide of the particular amino acid sequence. A source of indicator material is provided and is used to visually indicate that the particular amino acid sequence has been formed. Where less than three codons are used a single or di-peptide is formed while where at least three codons are used, a polypeptide amino acid sequence is formed.

In a like manner, according to yet another aspect of the invention, a method is provided for demonstrating the particular codon(s) which code for a particular amino acid sequence. Thus, using the method of the invention, mRNA-free reticulocyte lysate is incubated together with codon-containing polynucleotide which comprises at least one, and preferably, at least three codons, which code for the particular amino acid sequence. As a result of incubation, the particular amino acid is formed which is then visually indicated by means of an indicator material. Obviously, a preferred indicator material is one which is nonradioactive, and, is most preferably, an enzyme capable of inducing a color change in a dye.

## BRIEF DESCRIPTION OF THE DRAWINGS

Fig. 1 illustrates schematically an educational kit for illustrating the process of protein synthesis; and

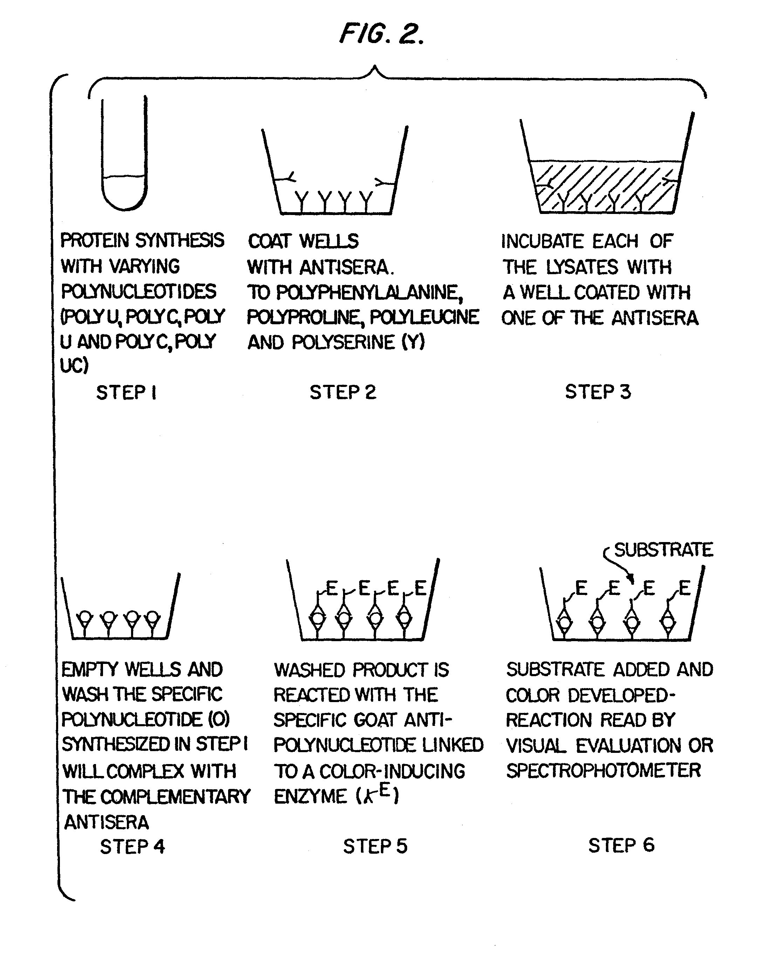

Fig. 2 illustrates an educational kit for demonstrating the particular codons used to produce a particular amino acid sequence.

## DESCRIPTION OF PREFERRED EMBODIMENTS

1. Educational Kit for Illustrating Process of Protein Synthesis

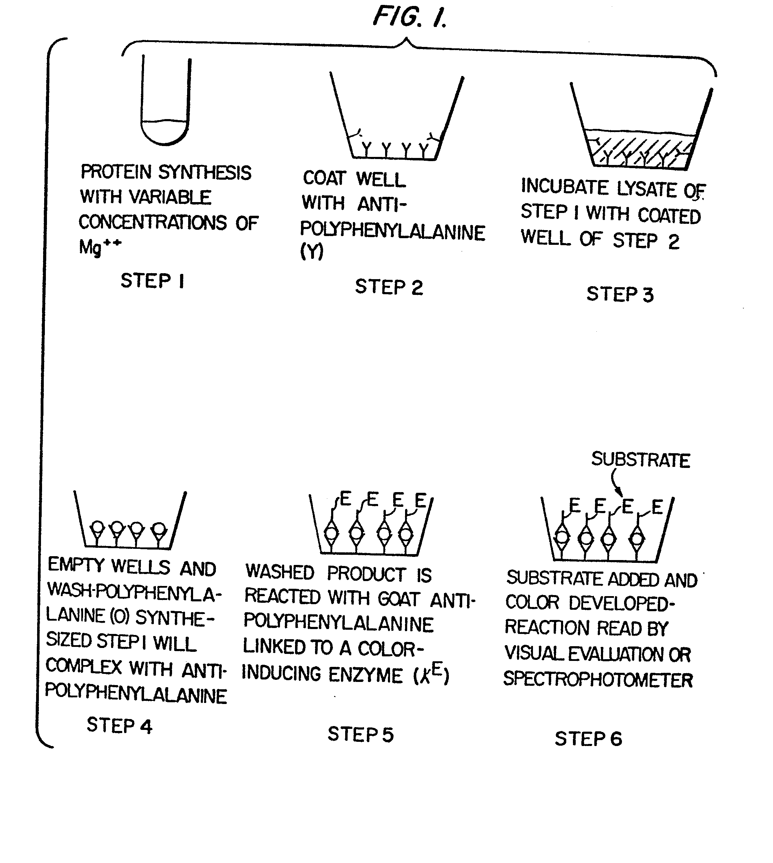

As may be seen with reference to Fig. 1, protein synthesis in a Micrococcus-treated reticulocyte lysate is performed in the presence of 5 micrograms/ml of polyuridilic acid. The synthesis is performed, preferably, using variable added quantities of Mg++, in concentrations from 0-15mM (final concentration), in test-tubes which contain a final volume of 100 microliters at a pH of about 7.4 and substrate conditions as in Pelham, R.B. and Jackson, R.J., Eur. J. Biochem, 67:247-256, 1976 (step 1). After incubation at about 30 deg. C for about 30 minutes, the solution is added to a microtiter plate in the presence of 15mM EDTA to block further Mg++ions (step 3). The wells are coated with goat antipolyphenylalanine (step 2). After an additional 30-60 minutes of incubation at 37 deg. C in the wells of the microtiter plate, the wells are emptied. The product polyphenylalanine adheres to the well walls and is washed twice with saline buffer solution containing detergent (step 4) so as to eliminate traces of the lysate. The washed product is then reacted with goat antipolyphenylalanine which is complexed with a color-inducing enzyme (step 5), e.g., horseradish peroxidase. If the two sets of antibodies used in steps 2 and 5 were both monoclonal antibodies, washing step 4 may be eliminated. The extent to which the antipolyphenylalanine has complexed with polyphenylalanine is indicative of the extent of polyphenylalanine which has been formed and can be readily observed by known enzyme-dye colorimetric techniques (step 6). The reaction may be further enhanced by the use of enzyme-labeled rabbit, donkey, or goat antibodies as a secondary reaction. Results are read either by visual evaluation of the staining reaction or by the use of a spectrophotometer.

2. Educational Kit for Demonstrating Particular Codons Used to Produce a Particular Amino Acid Sequence

Four lines of wells of a microtiter plate are each coated as follows:

a) one line of wells is coated with antipolyphenylalanine;

b) one line of wells is coated with antipolyproline;

c) one line of wells is coated with

- 6 -

0135279

antipolyleucine;

    d) one line of wells is coated with antipolyserine.

Protein synthesis in endogenous mRNA-free reticulocyte lysate is performed (step 1) in the presence of each of the four following starting materials:

    a) 5 micrograms of polyuridylic acid (poly U);

    b) 5 micrograms of polycytidylic acid (poly C);

    c) 2.5 micrograms of poly U and 2.5 micrograms poly C;

    d) Poly C, U, containing equal quantities of C and U.

Each of the starting materials is incubated as in example (1) for protein synthesis to occur, but in the presence of 5mM Mg++. Protein synthesis is then interrupted and the lysates are inactivated by adding 15mM EDTA. The proteins are then reacted with the rows of microtiter coated wells in the same manner as in Example (1) (steps 2 and 3). After amplifying by reaction with a second antibody, as was discussed in connection with Example (1) (steps 4-6), the following observations are made in connection with each of the syntheses:

Only antipolyphenylalanine-coated wells develop a staining reaction in connection with possibility (1), thus establishing that one of the codons for polyphenylalanine is UUU.

Antipolyproline-coded wells develop a staining reaction with possibility (2), thus establishing that one of the codons for proline is CCC.

With the mixture of possibility (3), the antipolyphenylalanine and antipolyproline-coated wells react, while neither antipolyserine nor antipolyleucine wells react. This again confirms that one of the codons for phenylalanine is UUU and that one of the codons for proline is CCC.

Using possibility (4), all of the coated wells develop a reaction, thus confirming the hybrid nature of the codons which cause the translation of phenylalanine, serine, leucine and proline. However, the antipolyserine and antipolyleucine-coated wells developed the strongest stained reaction confirming the alternating nature of the bases within the codons for each of these peptides.

The preceding results indicate the following requirements:

a) to produce polyphenylalanine the polynucleotide must be either polyuridylic acid homopolymer (UUU) or polyuridylicytidylic acid copolymer (UUC);

b) to produce polyserine the polynucleotide must be polyuridylicytidylic acid copolymer (UCU or UCC);

c) to produce polyleucine the polynucleotide must be polyuridylicytidylic acid copolymer (CUU or CUC); and

d) to produce polyproline the polynucleotide must be either polycytidylic acid homopolymer (CCC) or polyuridylicytidylic acid copolymer (CCU).

The above kits are merely examples of ways in which the principles of the invention may be achieved, i.e., laboratory kits which do not use radioactive-based indicators. Therefore, it is clear that the kits and method of the invention may undergo numerous variations without departing from the scope of the invention as defined by the claims.

WHAT IS CLAIMED IS:

1. An educational kit for illustrating the process of protein synthesis which is not radioactive-based comprising:

a) means for forming a homopolypeptide from a nucleotide source;

b) means for complexing said homopolypeptide with an anti-homopolypeptide antibody from an antibody source; and

c) means for complexing said homopolypeptide with an enzyme, said enzyme being capable of inducing a color reaction.

2. The educational kit as defined by claim 1 further comprising a polynucleotide source.

3. The educational kit as defined by claim 1 wherein said means for forming said homopolypeptide comprises a Micrococcus-treated reticulocyte lysate.

4. The educational kit as defined by claim 1 further comprising a supply of and means for adding different concentrations of Mg++ during formation of said homopolypeptide, whereby the role of Mg++ in protein synthesis may be demonstrated.

5. The educational kit as defined by claim 1 further comprising a microtiter plate with wells coated with said anti-homopolypeptide in which said homopolypeptide is placed.

6. The educational kit as defined by claim 1 wherein said means for complexing said homopolypeptide with said enzyme comprises an anti-homopolypeptide antibody with a color-inducing enzyme associated therewith.

7. The educational kit as defined by claim 6 wherein said enzyme is horseradish peroxidase.

8. An educational kit for demonstrating the particular codons used to produce a particular amino

0135279

acid sequence, said kit comprising:

a) a source of mRNA-free reticulocyte lysate;

b) a source of codon-containing polynucleotide comprising at least one codon which codes for said particular amino acid sequence;

c) means for incubating a mixture of said source materials of steps a) and b) to form a product polypeptide of said particular amino acid sequence; and

d) a source of indicator material used to visually indicate that said particular amino acid sequence has been formed.

9. The educational kit as defined by claim 8 wherein the codon-containing polynucleotide of step b) comprises at least three codons which code for said particular amino acid sequence.

10. The educational kit as defined by claim 8 wherein said indicator material comprises an antibody of said particular amino acid sequence.

11. The educational kit as defined by claim 10 further comprising a source of secondary antibody complexed with an enzyme, said secondary antibody being selected to complex with said particular amino acid sequence and/or said antibody whereby said enzyme can activate a color reaction.

12. The educational kit as defined by claim 8 wherein said source of indicator material is coated on the interior of a well of a microtiter plate.

13. The educational kit as defined by claim 8 wherein said poarticular amino acid sequence is polyphenylalanine and said codon-containing polynucleotide is selected from the group consisting of polyuridylic acid homopolymer (UUU) and polyuridylicytidylic acid copolymer (UUC).

14. The educational kit as defined by claim 8 wherein said particular amino acid sequence is polyserine, and said codon-containing polynucleotide is polyuridylicytidylic acid copolymer (UCU or UCC).

15. The educational kit as defined by claim 8 wherein said particular amino acid sequence is polyleucine and said codon-containing polynucleotide is polyuridylicytidylic acid copolymer (CUU or CUC).

16. The educational kit as defined by claim 8 wherein said particular amino acid sequence is polyproline, and said codon-containing polynucleotide is selected from the group consisting of polycytidylic acid homopolymer (CCC) and polyuridylicytidylic acid copolymer (CCU).

17. A method for demonstrating the process of protein synthesis which is not radioactive based, said method comprising the steps of:

a) forming a homopolypeptide from a polynucleotide source; and

b) complexing said homopolypeptide with an anti-homopolypeptide antibody from an antibody source and with an enzyme capable of inducing a color reaction.

18. The method of demonstrating the process of protein synthesis as defined by claim 17 comprising forming said homopolypeptide with a Micrococcus-treated reticulocyte lysate.

19. The method of demonstrating the process of protein synthesis as defined by claim 17 further comprising demonstrating the process with a plurality of test runs and adding different concentrations of Mg++ during formation of said homopolypeptide during successive runs to illustrate the role of Mg++ concentration in protein synthesis.

20. The method of demonstrating the process of protein synthesis as defined by claim 17 comprising complexing said homopolypeptide with anti-homopolypeptide antibody having a color-inducing enzyme associated therewith.

21. The method for demonstrating the process of protein synthesis as defined by claim 20 further comprising affixing said homopolypeptide of step a) to anti-homopolypeptide affixed to the walls of a microtiter plate whereby a sandwich is formed as a result of step b).

22. The method for demonstrating the process of protein synthesis as defined by claim 17 wherein said enzyme is horseradish peroxidase.

23. A method of demonstrating the particular codons used to produce a particular amino acid sequence, said method comprising the steps of:

a) incubating a mixture of mRNA-free reticulocyte lysate with a codon-containing polynucleotide which contains at least one codon which codes for said particular amino acid sequence; and

b) complexing said particular amino acid sequence with an indicator material so as to permit observation that said particular amino acid sequence has been formed by said codon.

24. The method for demonstrating the particular codons used to produce a particular amino acid sequence as defined by claim 23 wherein said indicator material comprises an antibody of said particular amino acid sequence.

25. The method for demonstrating the particular codons used to produce a particular amino acid sequence as defined by claim 23 further comprising complexing said particular amino acid with a secondary antibody complexed with an enzyme whereby said enzyme can activate a color reaction.

26. The method for demonstrating the particular codons used to produce a particular amino acid sequence as defined by claim 23 wherein said particular amino acid sequence is polyphenylalanine and said codon-containing polynucleotide is selected from the group consisting of polyuridylic acid homopolymer (UUU) and polyuridylicytidylic acid co-polymer (UUC).

27. The method for demonstrating the particular codons used to produce a particular amino acid sequence as defined by claim 23 wherein said particular amino acid sequence is polyserine, and said codon-containing polynucleotide is polyuridylicytidylic acid copolymer (UCU or UCC).

28. The method for demonstrating the particular codons used to produce a particular amino acid sequence as defined by claim 23 wherein said product polypeptide is polyleucine and said codon-containing polynucleotide is polyuridylicytidylic acid copolymer (CUU or CUC).

29. The method for demonstrating the particular codons used to produce a particular amino acid sequence as defined by claim 23 wherein said polypeptide is polyproline and said codon-containing polynucleotide is selected from the group consisting of polycytidylic acid homopolymer (CCC) and polyuridylicytidylic acid copolymer(CCU).

## FIG. I.

PROTEIN SYNTHESIS
WITH VARIABLE
CONCENTRATIONS OF
Mg$^{++}$

STEP I

COAT WELL
WITH ANTI-
POLYPHENYLALANINE
(Y)

STEP 2

INCUBATE LYSATE OF
STEP I WITH COATED
WELL OF STEP 2

STEP 3

EMPTY WELLS AND
WASH-POLYPHENYLA-
LANINE (O) SYNTHE-
SIZED STEP I WILL
COMPLEX WITH ANTI-
POLYPHENYLALANINE

STEP 4

WASHED PRODUCT IS
REACTED WITH GOAT ANTI-
POLYPHENYLALANINE
LINKED TO A COLOR-
INDUCING ENZYME ($X^E$)

STEP 5

SUBSTRATE

SUBSTRATE ADDED AND
COLOR DEVELOPED-
REACTION READ BY
VISUAL EVALUATION OR
SPECTROPHOTOMETER

STEP 6

## FIG. 2.

PROTEIN SYNTHESIS
WITH VARYING
POLYNUCLEOTIDES
(POLY U, POLY C, POLY
U AND POLY C, POLY
UC)

STEP I

COAT WELLS
WITH ANTISERA.
TO POLYPHENYLALANINE,
POLYPROLINE, POLYLEUCINE
AND POLYSERINE (Y)

STEP 2

INCUBATE EACH OF
THE LYSATES WITH
A WELL COATED WITH
ONE OF THE ANTISERA

STEP 3

EMPTY WELLS AND
WASH THE SPECIFIC
POLYNUCLEOTIDE (O)
SYNTHESIZED IN STEP I
WILL COMPLEX WITH
THE COMPLEMENTARY
ANTISERA

STEP 4

WASHED PRODUCT IS
REACTED WITH THE
SPECIFIC GOAT ANTI-
POLYNUCLEOTIDE LINKED
TO A COLOR-INDUCING
ENZYME ($\prec^E$)

STEP 5

SUBSTRATE

SUBSTRATE ADDED AND
COLOR DEVELOPED-
REACTION READ BY
VISUAL EVALUATION OR
SPECTROPHOTOMETER

STEP 6